(19) Europäisches Patentamt
European Patent Office
Office européen des brevets

(11) **EP 4 420 614 A1**

(12) **EUROPEAN PATENT APPLICATION**

(43) Date of publication:
**28.08.2024 Bulletin 2024/35**

(21) Application number: **23162534.4**

(22) Date of filing: **17.03.2023**

(51) International Patent Classification (IPC):
**A61B 8/08** (2006.01)    **A61B 8/00** (2006.01)

(52) Cooperative Patent Classification (CPC):
**A61B 8/0841; A61B 8/463; A61B 8/5215;**
A61B 8/4427; A61B 8/4488; A61B 8/468;
A61B 8/483; A61B 8/565; G01S 15/8915;
G01S 15/8997

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.02.2023 US 202363447775 P**

(71) Applicant: **Koninklijke Philips N.V.
5656 AG Eindhoven (NL)**

(72) Inventor: **ADAMS, Darwin Philip
Eindhoven (NL)**

(74) Representative: **Philips Intellectual Property &
Standards
High Tech Campus 52
5656 AG Eindhoven (NL)**

(54) **OBJECT LOCALIZATION / VISUALIZATION**

(57)     A method (100) for object (e.g. needle) location using ultrasound, comprising: (i) transmitting (120), from an element of an array transducer of an ultrasound system (200), a wide beam; (ii) receiving (130), at the array transducer, echo signals from the transmitted wide beam, wherein at least some of the echo signals are reflected from a needle located at least partially inside the patient; (iii) analyzing (140), by a processor of the ultrasound system, the received echo signals; (iv) determining (150), based on the analysis, a location of the needle inside the patient relative to the array transducer; and (v) reporting (160), to an ultrasound operator via a user interface, the determined location of the needle inside the patient, comprising overlaying the determined needle location over an ultrasound image of the patient obtained of the determined needle location.

FIG. 3

**Description**

FIELD OF THE INVENTION

The present disclosure is directed generally to methods and systems for object (e.g. **needle) localization and visualization using ultrasound.**

BACKGROUND OF THE INVENTION

Needles and other small objects are often inserted into an animal during a procedure such as a biopsy. When these objects are introduced into the animal, the location of the object is essential information in order to target or avoid parts of the body. Ultrasound is commonly used for needle guidance because it makes real-time images and gives good visibility to both the needle and the surrounding tissues.

Needle guidance using ultrasound requires intermediate to advanced clinical skills. For example, it requires hand-eye coordination, as the operator is typically using one hand to manipulate the needle and the other hand to manipulate the ultrasound transducer. A third hand would be useful to adjust the ultrasound machine settings but would require advanced UI controls to accomplish. Needle guidance also requires knowledge of the vulnerability and location of tissues and organs along the planned path of the needle. Needle guidance must also account for patient movement, which might move the needle and/or internal tissues. Needle guidance also requires knowledge of the exact location of the needle tip, as this is where materials are delivered and/or removed by the needle.

While ultrasound has been used successfully to image both a needle and surrounding tissue using focused and steered beams, a common problem with this design is that the needle can be hard to visualize unless the beams are nearly perpendicular the needle. This can again require significant effort, expertise, and time on the part of the operator to find the needle with the beam, typically involving adjustment of the transducer until the needle is visualized, and/or steering the image to various angles to select the view that best images the needle.

SUMMARY OF THE INVENTION

Accordingly, there is a continued need for ultrasound methods and systems for object (e.g. needle) localization and visualization using ultrasound. The invention is defined by the independent claims. The dependent claims define advantageous embodiments.

Accordingly, various embodiments and implementations herein are directed to an ultrasound method and system configured for object (e.g. needle) localization and visualization. The system transmits a wide beam from an element of an array transducer, and receives, at the array transducer, echo signals from the transmitted wide beam. A processor of the ultrasound system analyzes the received echo signals and determines, based on the analysis, a location of the needle inside the patient relative to the array transducer. The system then uses a user interface to report the determined location of the needle inside the patient, comprising overlaying the determined needle location over an ultrasound image of the patient obtained of the determined needle location. A wide beam in the meaning of this specification is any unfocused beam that is used to detect reflections from an object (e.g., a needle). The beam width may for example be 30 degrees, 40 degrees, 50 degrees, 60 degrees or 70 degrees, or any other beam angle between 30 and 70 degrees. Nonetheless, also larger and smaller beam widths may be used as long as the beam width is wide enough so as not to focus the beam on a particular feature.

Generally, in one aspect, a method for object (e.g. needle) location using ultrasound is provided. The method includes: (i) transmitting, from an element of an array transducer of an ultrasound system, a wide beam; (ii) receiving, at the array transducer, echo signals from the transmitted wide beam, wherein at least some of the echo signals are reflected from a needle located at least partially inside the patient; (iii) analyzing, by a processor of the ultrasound system, the received echo signals; (iv) determining, based on the analysis, a location of the needle inside the patient relative to the array transducer; and (v) reporting, to an ultrasound operator via a user interface, the determined location of the needle inside the patient, comprising overlaying the determined needle location over an ultrasound image of the patient obtained of the determined needle location.

According to an embodiment, overlaying the determined needle location over the ultrasound image comprises overlaying an image of a needle.

According to an embodiment, the method further includes determining, based on the determined location of the needle inside the patient, that the transducer array is at an improper orientation, and wherein reporting the determined location of the needle comprises issuing a warning.

According to an embodiment, the location of the needle inside the patient is determined in 3D space.

According to an embodiment, analyzing the received echo signals comprises a Hough Transform of the received echo signals, and/or comprises a perspective transformation of the received echo signals.

According to an embodiment, the needle is curved.

According to an embodiment, the wide beam is transmitted from a plurality of elements of the array transducer of the ultrasound system.

According to an embodiment, the method further includes adjusting the location of the needle in the patient based on the report of the determined location of the needle inside the patient. According to an embodiment, the location of the needle inside the patient is determined without steering or focusing of a transmitted beam.

According to another aspect, a computer program product comprises instructions which when executed by an object location system cause the system to carry out the steps of any above method.

According to another aspect, a method for guiding an object (e.g. needle) during an ultrasound of a patient is provided. The method includes receiving, at a user interface of an ultrasound system, a report comprising a determined location of the needle inside the patient, wherein the report is generated by a method for object location using ultrasound as described above (i.e.: (i) transmitting, from an element of an array transducer of the ultrasound system, a wide beam; (ii) receiving, at the array transducer, echo signals from the transmitted wide beam, wherein at least some of the echo signals are reflected from a needle located at least partially inside the patient; (iii) analyzing, by a processor of the ultrasound system, the received echo signals; (iv) determining, based on the analysis, a location of the needle inside the patient relative to the array transducer; (v) generating a report comprising the determined location of the needle inside the patient); and the method further includes adjusting the location of the needle in the patient based on the received report.

According to another aspect is a system for object location during an ultrasound of a patient, the system being configured to carry out any object location method described or claimed herein. The system may include a transducer probe configured to: (i) transmit, from an element of an array transducer, a wide beam; and (ii) receive echo signals from the transmitted wide beam, wherein at least some of the echo signals are reflected from a needle located at least partially inside the patient; a processor configured to: (i) analyze the received echo signals; and (ii) determine, based on the analysis, a location of the needle inside the patient relative to the array transducer; and a user interface configured to report the determined location of the needle inside the patient, comprising overlaying the determined needle location over an ultrasound image of the patient obtained of the determined needle location.

According to an embodiment, the processor is further configured to determine, based on the determined location of the needle inside the patient, that the transducer array is at an improper angle, and the user interface is further configured to issue a warning that the transducer array is at an improper angle.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.

Fig. 1 is a flowchart of a method for needle localization during an ultrasound, in accordance with an embodiment.
Fig. 2 is a schematic representation of an ultrasound system, in accordance with an embodiment.
Fig. 3 is a schematic representation of an ultrasound system transmitting a beam and receiving echo signals, in accordance with an embodiment.
Fig. 4A is an enhanced image of echoes from a linear array transducer imaging a needle embedded in an animal tissue, in accordance with an embodiment.
Fig. 4B is a prediction of the needle curve, in accordance with an embodiment.
Fig. 5 is a graph depicting needle depth measured using adjacent elements, in accordance with an embodiment.
Fig. 6 is a schematic representation of an ultrasound system transmitting a beam and receiving echo signals, in accordance with an embodiment.
Fig. 7A is a schematic representation of a 45deg needle angle, in accordance with an embodiment.
Fig. 7B is a schematic representation of a 22deg needle angle, in accordance with an embodiment.
Fig. 8 is a schematic representation of a linear array creating an image(s) by translating an aperture along the multiple array elements, in accordance with an embodiment.

Fig. 9 is a schematic representation of needle localization and analysis, in accordance with an embodiment.

Fig. 10 is a schematic representation of needle localization and analysis, in accordance with an embodiment.

Fig. 11A is a linear array image of tissue with two needles inserted, in accordance with an embodiment.

Fig. 11B is a divergent linear array image of the same tissue and two inserted needles from Fig. 11A, in accordance with an embodiment.

Fig. 12A is an ultrasound image of tissue comprising an inserted needle, in accordance with an embodiment.

Fig. 12B is an ultrasound image of tissue comprising an inserted needle, in accordance with an embodiment.

Fig. 12C is an ultrasound image of tissue comprising an inserted needle, in accordance with an embodiment.

Fig. 12D is an ultrasound image of tissue comprising an inserted needle, in accordance with an embodiment.

## DETAILED DESCRIPTION OF EMBODIMENTS

The present disclosure describes various embodiments of an ultrasound system and method configured to perform needle localization. **More generally,** Applicant has recognized and appreciated that it would be beneficial to provide improved ultrasound methods and systems for localization of a needle or other small object during a procedure. Accordingly, an ultrasound system transmits a wide beam from an element of an array transducer, and receives, at the array transducer, echo signals from the transmitted wide beam. A processor of the ultrasound system analyzes the received echo signals and determines, based on the analysis, a location of the needle inside the patient relative to the array transducer. The system then uses a user interface to report the determined location of the needle inside the patient, comprising overlaying the determined needle location over an ultrasound image of the patient obtained of the determined needle location.

According to an embodiment, the systems and methods described or otherwise envisioned herein can, in some non-limiting embodiments, be implemented as an element for a commercial product for ultrasound imaging or analysis, such as Philips® Affiniti®, or as an element for a commercial product for patient analysis or monitoring, such as the Philips Patient Flow Capacity Suite (PFCS), or any suitable system.

According to an embodiment, the systems and methods described or otherwise envisioned herein enable localization of a needle path over a wide range of needle/beam angles, and can properly show a needle path regardless of where the needle is inserted (i.e., left or right). The needle path is retrained despite small movements of the transducer or of the patient. Additionally, the systems and methods described or otherwise envisioned herein perform up to 100x faster than conventional techniques, which allows the main image of the body to run at full frame rate while tracking the needle path in real time. The systems and methods described or otherwise envisioned herein require neither focusing nor steering the ultrasound beams, which provides a simpler and more robust solution to the problems currently faced by ultrasound localization. In other words, an unfocused beam may be transmitted with a typical wide beam configuration, that is, with a beam angle of, for example, 30 degrees, 40 degrees, 50 degrees 60 degrees or 70 degrees, or any other beam angle between 30 and 70 degrees. Nonetheless, also wider and narrower beam angles can be used without deviating from the invention, as long as the beam angle is wide enough to not focus the image. The resulting beams will not focus well on fine tissues which may scatter the beam (e.g., Rayleigh scattering or Mei scattering), while an inserted object (e.g., the needle) will form a large "specular" reflection allowing it to be more prominent than fine tissues, thus enabling its identification and processing according to the invention.

According to an embodiment, the systems and methods described or otherwise envisioned herein uses divergent beams and avoids conventional point focused transmit and receive beamforming. According to one embodiment, a single element transmits a wide divergent beam and listens on all elements of the array simultaneously recording echoes. These echoes are then analyzed based on Snell's law which states that for flat or straight objects, the angle of incidence equals the angle of reflection. Geometrical calculations locate the needle point of entry relative to the end of the transducer array and the angle of the needle relative to the plane of the array. Thus, the system can localize needles over a wide range of angles and points without the limitations inherent in the prior art. For example, a needle can be localized with as little as a single transmit event and without the need to steer or focus the ultrasound beams.

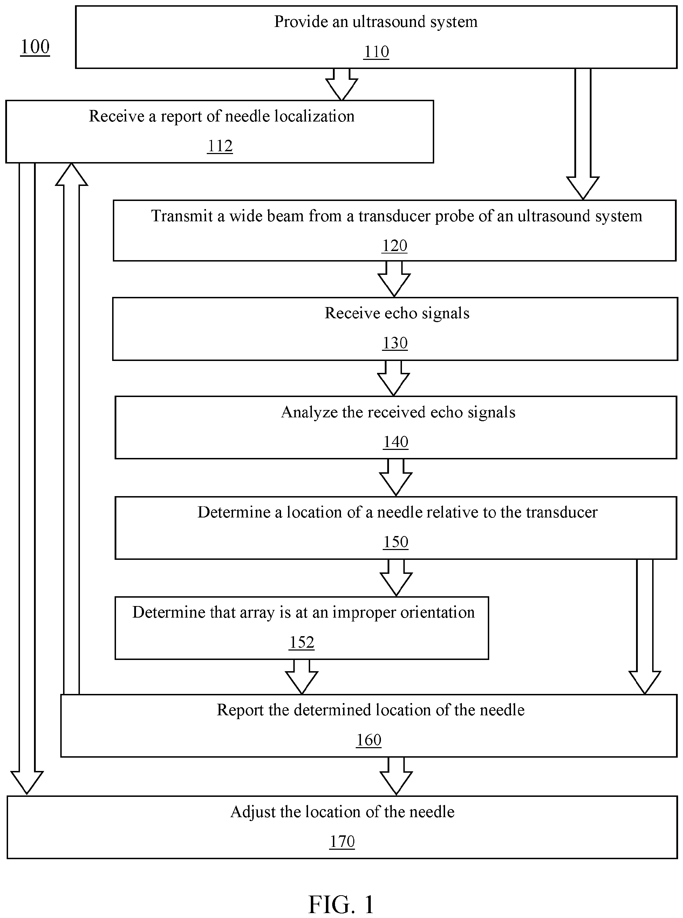

Referring to Fig. 1, in one embodiment, is a flowchart of a method 100 for needle localization using an ultrasound system. The methods **described in connection with the** figures are provided as examples only, and shall be understood to not limit the scope of the disclosure. The ultrasound system **can be any of the systems** described or otherwise envisioned herein. The ultrasound system **can be a single system or multiple different systems.**

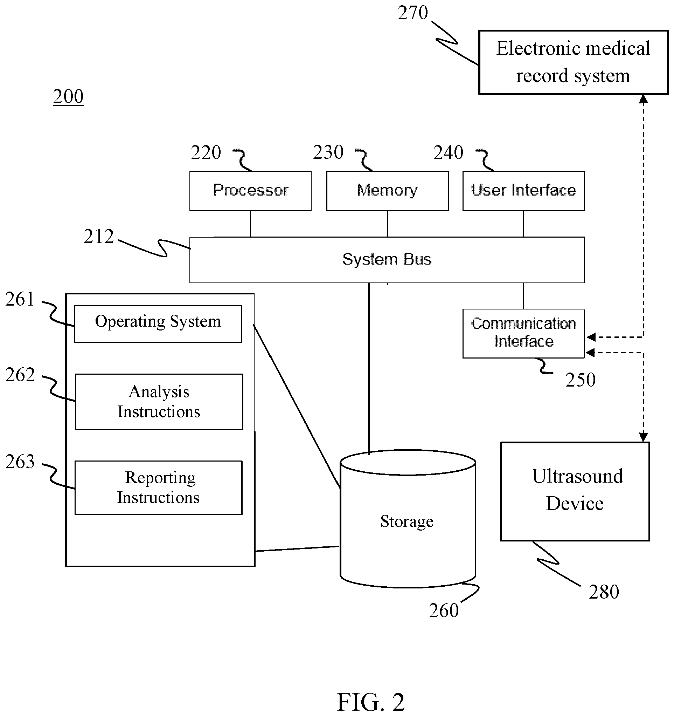

**At step 110 of the method, an** ultrasound system **200 is provided.** Referring to an embodiment of an ultrasound system 200 as depicted in Fig. 2, for example, the system comprises one or more of a processor 220, memory 230, user interface 240, communications interface 250, and storage 260, interconnected via one or more system buses 212. It will be understood that Fig. 2 constitutes, in some respects, an abstraction and that the actual organization of the components of the system 200 may be different and more complex than illustrated. Additionally, ultrasound system 200 can be any of the systems described or otherwise envisioned herein. Other elements and components of ultrasound system 200 are disclosed and/or envisioned elsewhere herein.

According to an embodiment, the ultrasound systems and methods described or otherwise envisioned herein are utilized to localize and/or visualize a needle or other small object inserted at least partially into an animal, such as a human. For example, the ultrasound systems and methods can be utilized to localize the placement of, and assist with guidance of, a needle used during a procedure such as a biopsy, medicine placement/insertion, and/or any other procedure or utilization.

At step 120 of the method, a transducer of the ultrasound system transmits a beam while the needle is at least partially inserted. According to an embodiment, a wide beam is transmitted from an element of an array transducer of the ultrasound system. The beam can be generated and transmitted according to any method for generating and transmitting beams from a transducer of an ultrasound. According to an embodiment, the **beam is transmitted from a plurality of elements of the array transducer of the ultrasound system, such as from two or more elements.** According to an embodiment, the **beam is transmitted with every frame.**

According to an embodiment, an operator manipulates a transducer probe during beam transmission. The operator of the transducer and ultrasound may be the same user manipulating the needle in the patient, or may be another individual. **The user may be any user capable of or authorized to perform an ultrasound, such as a sonographer, nurse, physician, emergency medical technician, paramedic, or any other individual.** According to an embodiment, the ultrasound device or system is a stationary device, a portable device, or a handheld device. **Many other** ultrasound devices and systems are possible.

**According to an embodiment, a plurality of ultrasound images are obtained of any portion of the subject. The** ultrasound image data may be obtained using any ultrasound device or system, which may be any device or system suitable to obtain or otherwise receive ultrasound image data of the patient. **The** ultrasound image data may be obtained as 2D or 3D data. **The** ultrasound image data may be obtained as video data. One or more parameters of the ultrasound device can be set, adjusted, preprogrammed, or otherwise determined by a healthcare professional. The ultrasound device or system comprises an **ultrasound transducer probe configured to obtain the ultrasound images.**

**According to an embodiment, the** ultrasound system may comprise patient data about the subject **for which a procedure and ultrasound localization will be performed. The** patient data can be any information about the patient **that the** ultrasound system **can or may utilize for analysis as described or otherwise envisioned herein. According to an embodiment, the patient** data **comprises one or more of demographic information about the patient, medical history of the patient, a diagnosis for the patient, and a reason for the ultrasound to be performed. For example, demographic information may comprise information about the patient such as name, age, body mass index (BMI), and any other demographic information. The medical history of the patient may be any historical admittance or discharge information, historical treatment information, historical diagnosis information, historical exam or imaging information, and/or any other information. The diagnosis for the patient may be any information about a medical diagnosis for the patient, historical and/or current. The reason for the ultrasound to be performed may be any purpose, reason, need, or other impetus for the exam.**

**The patient** data **is received from one or a plurality of different sources. According to an** embodiment, **the patient** data **is received from, retrieved from, or otherwise obtained from an electronic medical record (EMR) database or system. The EMR database or system may be local or remote. The EMR database or system may be a component of the** ultrasound system, **or may be in local and/or remote communication with the** ultrasound system. **The received patient data may be utilized immediately, or may be stored in local or remote storage for use in further steps of the method.**

**The needle or other small object can be anything utilized for a procedure which requires localization. For example, the needle or other small object may be a tool to deliver something such as a medicine or treatment to the body, and/or to retrieve something such as a sample from the body. The needle or other small object may be a straight object, or may be a curved object, along with other possible shapes and sizes.**

At step 130 of the method, the array transducer of the ultrasound system receives echo signals from the transmitted wide beam, where at least some of the echo signals are reflected from a needle located at least partially inside the patient. The echo signals can be received according to any method for receiving echo signals by a transducer of an ultrasound system.

According to an embodiment, a transmit/receive switch reduces receive overload during the transmit pulse. An analog time-gain ("TGC") can be applied to the received echo signals, and the signals can be digitized and provided to a processor for analysis. According to embodiment, no delay or summing is done with the received echo signals.

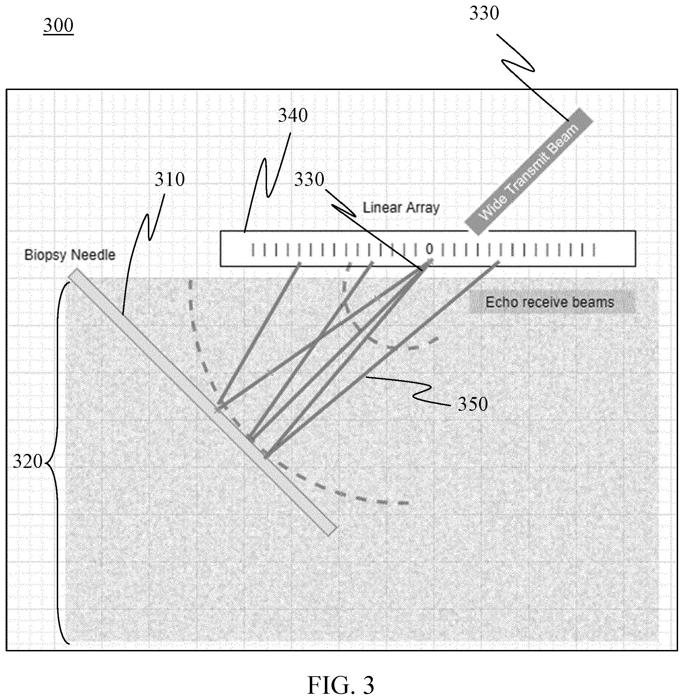

Referring to Fig. 3, in one embodiment, is a schematic representation 300 of an ultrasound system transmitting a beam and receiving echo signals while the needle is at least partially inserted. In the representation, a needle 310 is at least partially inserted into a tissue 320 of an animal or individual. A transducer of the ultrasound system transmits a wide transmit beam 330 while the needle is at least partially inserted. The linear array 340 of the ultrasound system receives the echo signals 350, at least some of which are reflected from the needle 310. The beam and echo pairs obey Snell's law with the angle of incidence equal to the angle of reflection.

At step 140 of the method, a processor of the ultrasound system analyzes the received echo signals. According to an embodiment, the processor analyzes each channel individually, finds patterns in the echoes that match a model, and selects the pattern that best matches the model. According to an embodiment, when analyzing each channel individually, the system can bandpass the signal with 30% bandwidth at a center frequency of the transmit waveform. The system can apply the TGC with the same properties utilized for the main image. The system can detect the waveform using a Hilbert transform or equivalent circuit.

According to an embodiment, when finding a pattern or patterns in the echoes that match a model, the system can search the receive waveform for peaks and create a new waveform with only the peaks and their time locations. Peaks will be found in the waveform when an echo is received from an object such as a needle.

According to an embodiment, when selecting a pattern that best matches the model, the system utilizes a model that relates echo time location to needle angle, and an offset is used to create a Hough Transform of the received data. The needle angle and offset is found by searching the transform for its peak value.

The results of the analysis by the processor **may be utilized immediately, or may be stored in local or remote storage for use in further steps of the method.**

**At step 150 of the method the system determines, based on the analysis by the processor,** a location of the needle inside the animal or individual relative to the array transducer. Notably, the location of the needle inside the patient is determined without steering or focusing of the transmitted beam.

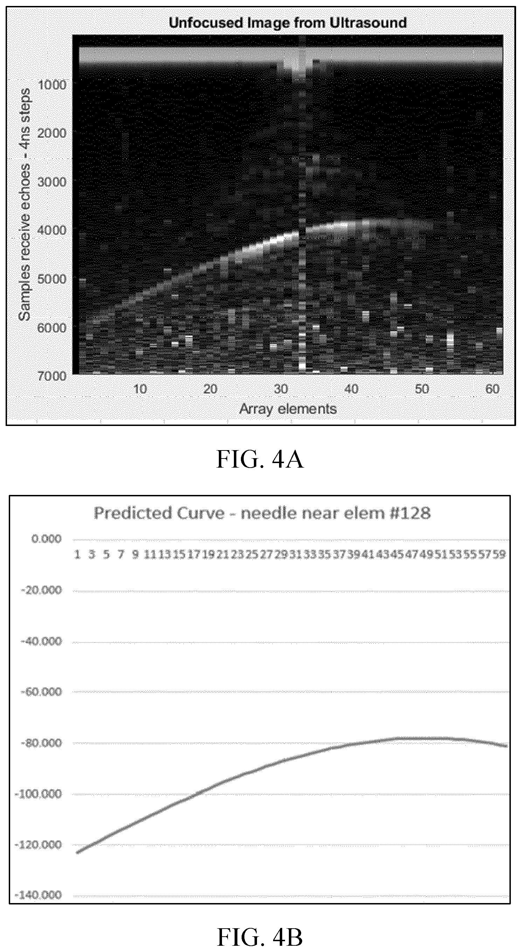

Referring to Figs. 4A and 4B, in one embodiment, is an example of needle localization using the methods and systems described or otherwise envisioned herein. For example, referring to Fig. 4A is an enhanced image of echoes from a linear array transducer imaging a needle embedded in an animal tissue. There is a long lateral curve of the needle from echoes on each element. Referring to Fig. 4B is a prediction of the curve using a model according to the methods and systems described or otherwise envisioned herein.

According to an embodiment, at optional step 152 of the method, the system determines, based on the determined location of the needle inside the patient, that the transducer array is at an improper orientation, such as if the needle is being inserted at an unexpected end of the array. Inserting the needle at the wrong location or at a location on the system screen that is unexpected could be a threat to patient safety. For example, a possible mistake is to hold the array in the wrong orientation which can surprise the user when the needle is not visible due to this error. The methods and systems described or otherwise envisioned herein detect the needle path regardless of the left/right orientation of the transducer. Since many ultrasound systems have a left-right control that determines which end of the array is displayed at the left of the image on the screen, the system can issue a warning when the orientation does not match the needle path.

At step 160 of the method the system provides or reports, to an ultrasound operator via a user interface, the determined location of the needle inside the animal or patient. According to an embodiment, the reported location comprises overlaying the determined needle location over an ultrasound image of the patient obtained of the determined needle location. For example, as just one non-limiting example, overlaying the determined needle location over the ultrasound image comprises overlaying an image of a needle. The image may be an actual image of the needle, may be a CGI-like representation of a needle, or may be any other image used to represent the needle.

The determined location can be provided or reported to the user via any known mechanism for providing object location information. For example, object location information may be provided on an ultrasound exam window, in another window or on another screen, as a visual display such as a projector or a wearable device, and via any other mechanism. The provided object location information can comprise any of the elements of the analysis, and may optionally include other information such as patient demographics or medical information, and/or any other information. As an example, the object location information can be displayed to the user via the user interface as one or more labeled images.

According to an embodiment, method 100 for needle localization using an ultrasound system comprises determining a location of the needle inside the animal or individual, and providing that information to a user. Thus, according to one embodiment, at step 112 of the method an operator or user receives a report comprise the location of the object, where the location of the object is determined as described above.

At step 170 of the method, the user or operator utilizes the received location information. The user or operator may utilize the received location information in a wide variety of ways. For example, according to one embodiment, the user or operator may determine based on the report of location information that the needle is in the proper orientation, angle, and/or location. According to another embodiment, the user or operator may determine based on the report of location information that the needle is an improper orientation, angle, and/or location. Thus, the user or operator will adjust the orientation, angle, and/or location of the needle. New image data can then be obtained, and the new location of the needle can be obtained. Many other ways to utilize the reported needle location information are possible.

Many variations of the methods and systems described or otherwise envisioned herein are possible. For example, for a system that steers to create a needle image, the needle path is used by the system to optimize its steering and its needle image. The needle path is very responsive and reliable, but typically does not show the actual needle, its parts, and the actions near the tip such as fluid exit or withdrawal. Using the path to control needle imaging combines the advantages of the path with the clinical need to see the needle image.

According to an embodiment, the ultrasound system may comprise special purpose or 3D/4D arrays can track the needle more completely. Typical transducers often have a fixed elevation aperture and focus that creates a narrow beam in the plane orthogonal to the imaging plane. However, transducers such as 3D/4D arrays can also visualize in the elevation plane. Thus, the needle path algorithm can be extended to include needles in 3D space instead of just 2D space. Conventional focused imaging algorithms may fail to visualize a needle in 3D because of the same perpendicular issues discussed herein in the 2D plane. However, the methods and systems described or otherwise envisioned herein solves this in 3D and therefore can give a much better visualization of the needle path when compared to conventional designs.

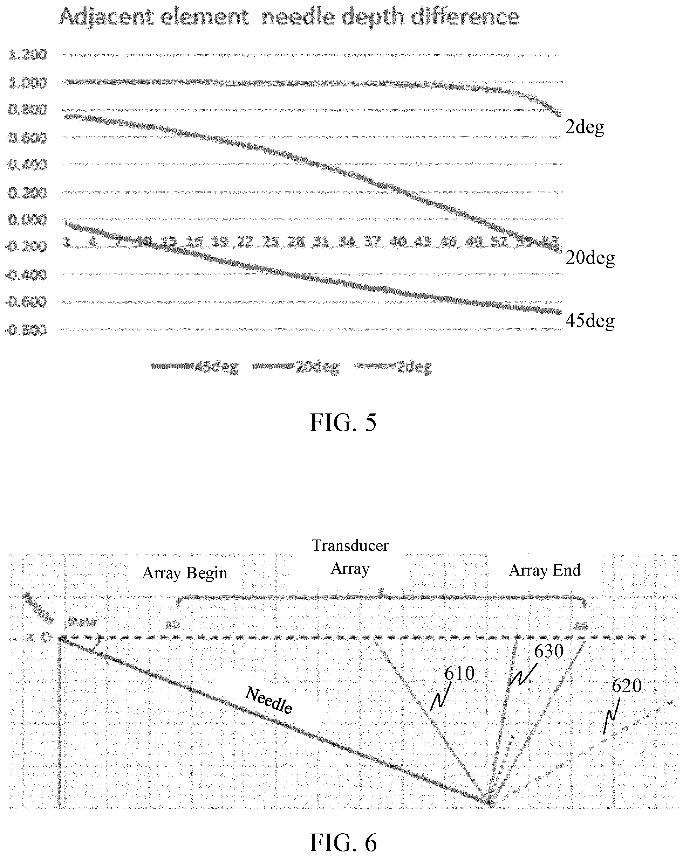

According to an embodiment, the ultrasound system utilizes a Hough Transform to extract the needle path from the received data. Alternatives to a Hough Transform are also possible. In some embodiments, a Hough Transform can take a long time to compute its solution, as the transform finds any pattern in the data that matches the model and searches all combinations. Ultrasound images are not symmetrical. Echoes are not created in the lateral dimension, only in the axial dimension. While it is it is easy to image a needle oriented in the lateral dimension, it is extremely difficult to image the same needle oriented in the axial dimension. Adjacent element information may be utilized as an alternative to Hough Transform, and can be faster and more accurate than the Hough Transform method. Referring to Fig. 5, in one embodiment, is a graph depicting needle depth measured using adjacent elements.

According to an embodiment, the ultrasound system creates an image from the needle path information. Both the needle path and needle image can be important information for the clinician. One approach is to use the path information to steer a conventional image. However, the system can also create images from the needle path information. According to one embodiment, conventional beamforming such as delay and sum can be applied to the element signals. Since only a single transmit might be utilized, the system may not get the benefits of transmit beamforming. However, an image can still be created.

According to another embodiment, the system can utilize synthetic aperture beamforming. In this case several divergent transmit beams can be used, up to a maximum of the number of channels in the array. Synthetic aperture techniques can be used to create a fully focused image in both transmit and receive, which might be an ideal situation for objects that are not moving. However, even small motions can ruin a synthetic aperture images. Regardless, there is flexibility in this method, as there is in conventional imaging, to trade of image quality and frame rate.

According to another embodiment, the system can optimize transmit/receive options to create an image from the needle path information. While a preferred embodiment utilizes a single transmit element to locate the needle, beamformed transmits from several elements can be included in this invention. Variations on the transmit aperture and waveform can be made to work with the methods and systems described or otherwise envisioned herein.

The methods and systems described or otherwise envisioned herein may be utilized to localize a target other than a straight needle. For example, since the methods and systems utilize divergent beams and track the echoes without conventional beamforming focusing, the techniques can be used to find many variations including curved needles. The important aspect is that the shape of the target is well defined and can be modelled.

The methods and systems described or otherwise envisioned herein may be utilized with multiple element apertures. As described herein, a preferred embodiment utilizes a single transmit element and single receive elements. However, any group of one or more elements could be used. So, for example, the adjacent transmit or receive elements could still work.

The methods and systems described or otherwise envisioned herein may be utilized with multiple transmits. As described herein, a preferred embodiment utilizes a single transmit event. However, multiple transmits may be used to improve performance. According to an embodiment, signal-to-noise ratio (SNR) can be improved by repeated transmits due to averaging of noise during reception. Further, multiple transmit locations may provide better coverage. For example, the system can transmit at the first element, the center element, and the last element giving the system a wider field of view. Referring to Fig. 6, in one embodiment, a center element transmitted beam 610 cannot identify the end of the needle tip (shown by the center transmit echo 620 missing the end of the array). However, the right end of the array easily identifies/localizes the end of the needle tip.

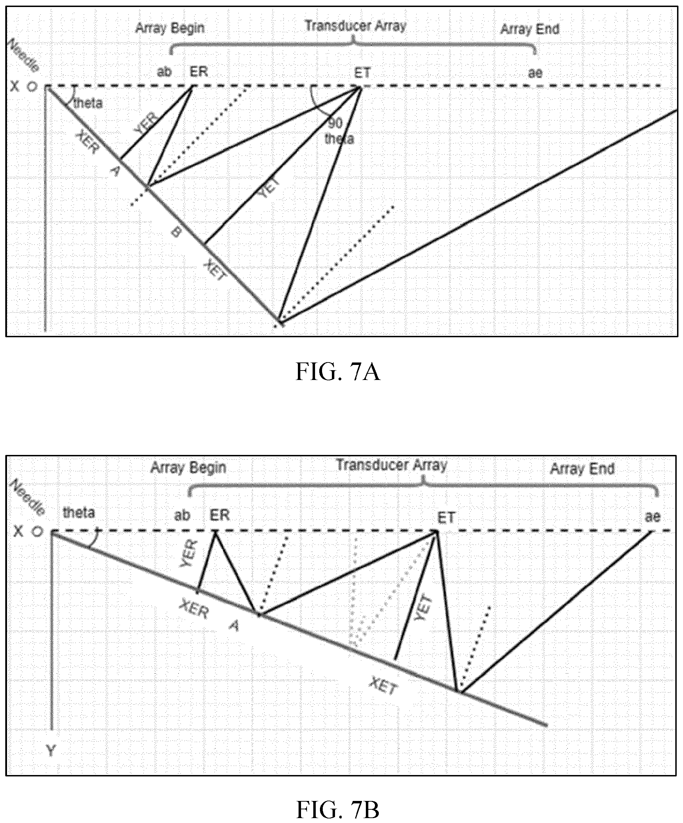

The methods and systems described or otherwise envisioned herein may be utilized with receive element delays. According to an embodiment, element delays may be used to steer the receive toward the anticipated direction of reflected echoes. Referring to Figs. 7A and 7B are representations of 45deg (Fig. 7A) and 22deg (Fig. 7B) needle angles (where ET = transmit element(s)). The receive steering can be used to reject echoes at undesired angles and intensify reception from desired angles.

Referring again to Figs. 7A and 7B, in accordance with an embodiment, the x-axis beings at the puncture origin "O" of the needle. All transducer elements in this embodiment also lie along the x-axis. According to an embodiment, the needle path is defined by two parameters: (i) the distance O2ab which is the distance between "O" and the beginning of the transducer array; and (ii) theta which is the angle of the needle measured from the x-axis.

According to an embodiment, there are three additional parameters that complete the model layout: (i) ER which is the receive array element; (ii) ET which is the transmit array element; and (iii) dist which is the distance travelled by ultrasound from ET to ER via Snell's law reflecting from the needle.

According to an embodiment, the model comprises the following trigonometry:

$$XER = ER * \cos(theta), \text{ and } YET = ER * \sin(theta) \tag{Eq. 1}$$

$$XET = ET * \cos(theta), \text{ and } YET = ET * \sin(theta) \tag{Eq. 2}$$

Per Snell's law, there is a point along the needle where the angle from that point is the same to ER as it is to ET, and the distance from XER to that point is A. Note also that the right triangles to that point for ER and ET are similar, which simplifies the equation.

Next, according to an embodiment:

$$A = (XET\text{-}XER) / (1 + YET/YER) \quad (\text{similar triangles}) \tag{Eq. 3}$$

$$dist = \sqrt{(A^2 + YER^2)} * (1 + YET/YER) \tag{Eq. 4}$$

which leads to the following function (such as via Matlab):

$$\text{function dist\_model} = \text{ComputeDist}( ER, ET, Theta )$$

$$\text{dist\_model} = (1+ET/ER) * \sqrt{(ER^2 * \sin(Theta)^2 + (-ER*\cos(Theta) + ET*\cos(Theta^2))} / \ldots \quad ( 1+ET/ER)^2);$$

end

For the Hough Transformation, the input data domain is dist/ER. The Hough accumulator domain is Theta/O2ab, and the Hough accumulator can be filled as follows: (i) using a single transmit element ET; and (ii) for each peak in the input data: for each of 50 different angles of Theta, for each of 80 values of O2ab, the following:

$$\text{Dist\_model} = \text{ComputeDist}(ER,ET,Theta) \tag{Eq. 5}$$

The find the proximity which is:

$$\text{abs}( \text{disk} - \text{dist\_model}) \tag{Eq. 6}$$

According to an embodiment, if proximity is < 2 then increment the Accumulator(Theta, O2ab) where the size of the increment is proportional to the brightness of the peak.

The methods and systems described or otherwise envisioned herein may be utilized with focused transmits. As described herein, a preferred embodiment utilizes a small number of transmit elements with a wide beam to illuminate the entire needle. However, transmit focusing can be used to reduce the signals from unwanted directions. Fig. 6 shows the geometry of a sample transmit and receive. According to an embodiment, a transmit beam such as 630 could be focused away from the end of the needle thereby improving the signal strength and avoiding unwanted tissue.

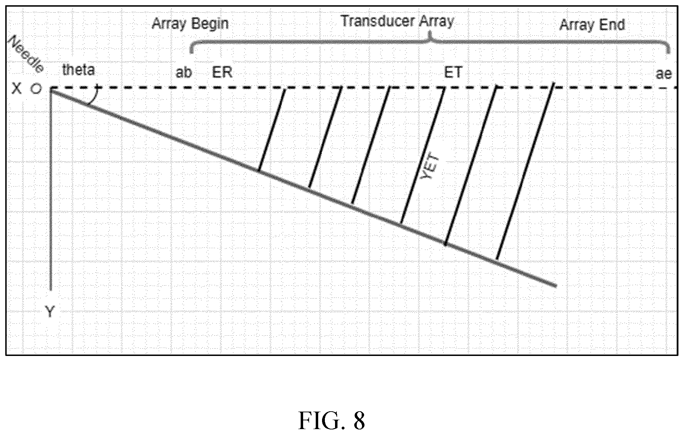

According to an embodiment, the methods and systems described or otherwise envisioned herein may be utilized with conventional ultrasound hardware and software. For example, the system could be used with linear and curved linear arrays that create images by translating an aperture along array elements. For example, referring to Fig. 8 the system could be utilized with a linear array to create an image(s) by translating an aperture along the multiple array elements.

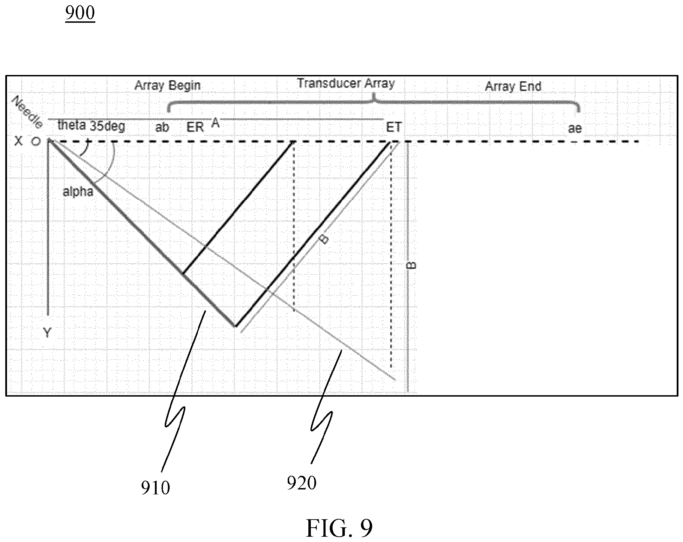

According to an embodiment, with reference to Fig. 9 is a schematic representation 900 of needle localization and analysis. An ultrasound system transmits a beam and receives echo signals while a needle 910 is at least partially inserted inside a tissue or other structure. A transducer of the ultrasound system transmits a wide transmit beam while the needle is at least partially inserted. The linear transducer array of the ultrasound system receives the echo signals, at least some of which are reflected from the needle 910. According to an embodiment, while line 910 represents the actual needle inserted in the tissue, line 920 represents how the ultrasound system detects the needle and how it would be displayed or represented on the ultrasound screen, As shown in Fig. 9, the representation 920 of the needle is not the actual location of the needle 910, and thus the received signal must be manipulated as described or otherwise envisioned herein such that an adjusted representation of the needle is the actual location of the needle 910.

According to an embodiment, using the cadence for a linear array, the number of transmit and receive elements is set to one (1). Thus, **each element will be used once per imaging frame. It will transmit and receive on its single element and produce one image line for the displayed image. For linear arrays, the system knows to form a full image from the lines that the acquisition system provides.**

**In accordance with an embodiment, the methods described or otherwise envisioned herein comprise an implementation in which needle visualization can be implemented on any ultrasound machine that supports linear arrays. Thus, rather than requiring new equipment or an equipment modification, a system can comprise software that implements the methods described or otherwise envisioned herein.**

**As shown in Fig. 9, echoes from the needle 910 at angle alpha will be displayed on the screen at angle theta. The system will display the echoes as though they were directly in front of the transducer elements, but the echoes normally come in at an angle. Accordingly, the perceived location 920 of the needle, or the viewed image provided by the system, must be converted to the actual location 910 of the needle. The methods described or otherwise envisioned herein make this conversion and identify which lines come from a needle rather than objects that are not valid needles. For example, the following equations are utilized to convert the perceived location 920 of the needle to the actual location 910 of the needle:**

$$B/A = \tan(\text{theta}) = \sin(\text{alpha}) \qquad\qquad \text{(Eq. 7)}$$

$$\text{alpha} = \arcsin(B/A) \qquad\qquad \text{(Eq. 8)}$$

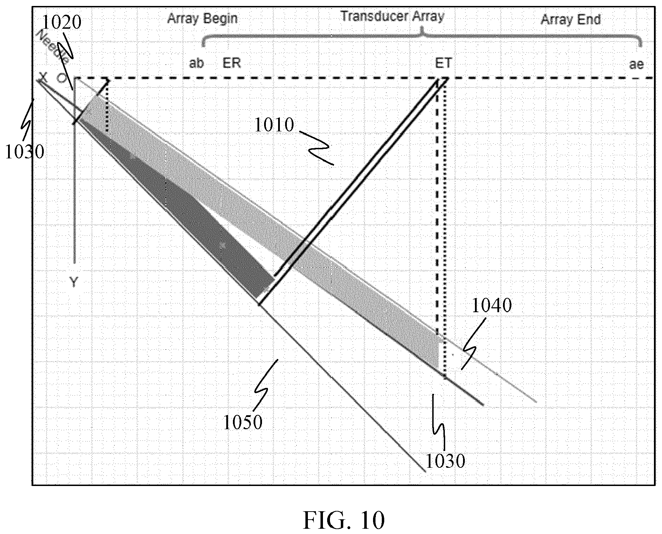

**According to an embodiment, needle visualization preferentially shows an image of the needle and not just its coordinates. Referring to Fig. 10, the length and thickness of a needle 1050 are distorted in a divergent beam image. The figure shows the proximal echo 1010 from the tip of the needle and a distal echo 1020 from the back of the needle. A different echo pattern for the needle, and a divergent beam image 1030 is generated rather than the expected perceived location 1040 of the needle shown in Fig. 9 (relative to the actual location 1050). Similar behavior can occur in photographs. For example, an image of a building taken from the bottom of the building looking upward will show distortion of the building based on the perspective of the camera. A perspective transform can correct the distortion.**

**In accordance with an embodiment, therefore, received echoes are processed with a perspective transform to correct the divergent beam image. This utilizes the divergent image directly, and corrects it to generate the correct image of the needle.**

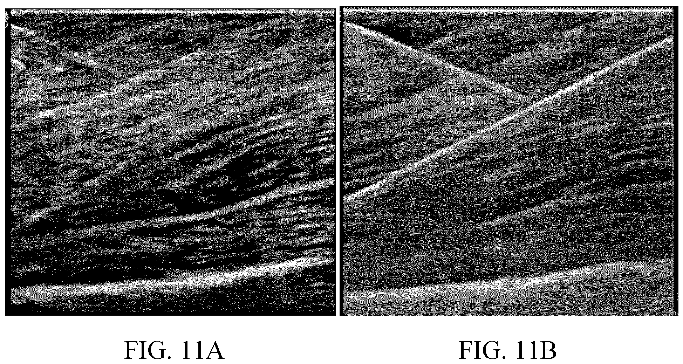

**Referring to Figs. 11A and 11B are ultrasound images of tissue comprising an inserted needle, without needle visualization processing. Fig. 11A is a normal linear array image of tissue with two needles inserted. Fig. 11B is a divergent linear array image of the same tissue and two inserted needles. According to an embodiment, image processing is required to suppress non-needle portions of the image such that the needle images stand out and can be properly utilized. Many needle visualization techniques work by steering focused beams orthogonal to the needle. In Fig. 11A the image is steered downward and has trouble seeing the needles. The divergent image in Fig. 11B finds needles simultaneously at all angles. While the divergent image in Fig. 11B suppresses non-needle shapes, it is still necessary to locate the best candidates for needles to display.**

**Thus, in accordance with an embodiment, the divergent image is analyzed to identify lines in the image that constitute a needle. The possible properties of a needle when imaged by divergent-beam acquisition are as follows. Some or all of these properties may be utilized for the image analysis:**

> **A very straight line (if the needle is straight);**
> **A thin line (most needles will not be thick, and the thickness can be a known parameter);**
> **The Point of Entry is outside of the array and constrained via limiting parameters (e.g. needle length, which can be a known parameter);**
> **The angle of the needle will be between 0 and 45 degrees;**
> **The needle will be brighter than non-specular echoes due to its hard and shiny surface;**

**The brightness will have a convex shape orthogonal to its track (some exceptions allowed);
It will have contiguous axial sections that satisfy the brightness properties, but can have other sections that do not;
Portions of the needle track may fail these properties, and the system can apply boundaries to this fact;
Portions that succeed these checks must lie on a thin, straight line (because the needle is straight);
Portions that fail must be a minority of the line; and/or
The end of the needle is defined as the last (furthest from the point of entry) where the criteria fail.**

According to an embodiment, the technique rotates the image to an angle, and then processes along horizontal lines. The processing accentuates needle properties.

Accordingly, once a divergent image is acquired, it can be processed as **described or otherwise envisioned herein utilizing a perspective transform to correct the divergent beam image and generate a true location of the needle, which can then be displayed to the user of the ultrasound system. This perspective transform can be accomplished utilizing any perspective transformation that is sufficient to properly localize a needle utilizing the** divergent image. The perspective transform can utilize some or all of the **properties enumerated in the list above.**

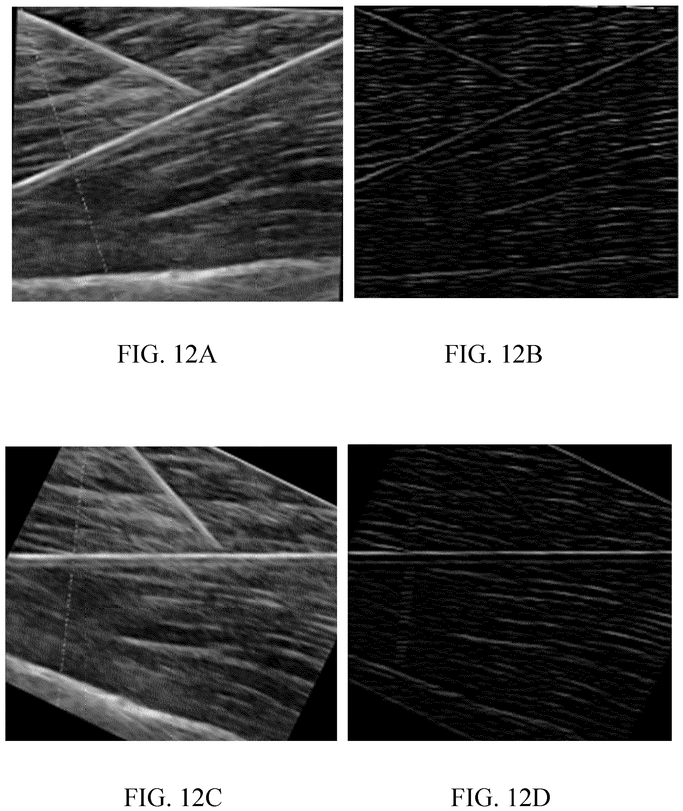

Referring to Figs. 12A-12D are **ultrasound images of tissue comprising an inserted needle, where Figs. 12A and 12C are divergent images, and Figs. 12B and 12D are images processed as described or otherwise envisioned herein. Thus, Fig. 12A is a divergent image, and Fig. 12B is the same image after the perspective transformation. Similarly, Fig. 12C is the divergent image, and Fig. 12D is the same image after the perspective transformation. In Figs. 12B and 12D, the needle is** clearly identified in the images. Figs. 12A and 12B are at zero degrees, and Figs. 12C and 12D are rotated to 20 degrees. In this implementation, the processed images are filtered for needles on horizontal lines.

Accordingly, at step 150 of the method where the system determines, based on the analysis by the processor, a location of the needle inside the animal or individual relative to the array transducer, this determination may comprise the perspective transformation as described or otherwise envisioned herein.

Referring to Fig. 2 is a schematic representation of an ultrasound system 200. System 200 may be any of the systems described or otherwise envisioned herein, and may comprise any of the components described or otherwise envisioned herein. It will be understood that Fig. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random-access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, the ultrasound system comprises or is in communication with an electronic medical record system 270 which is an electronic medical records database from which the information about the patient, including clinical information and ultrasound data, may be obtained or received. The electronic medical records database may be a local or remote database and is in direct and/or indirect communication with the ultrasound system 200. Thus, according to an embodiment, the ultrasound system comprises an electronic medical record database or system 270.

According to an embodiment, the system comprises one or more ultrasound devices 280 capable of acquiring the required ultrasound images or analysis. According to another embodiment, ultrasound system 200 is in wired and/or wireless communication with a local or remote ultrasound device 280 capable of acquiring the required ultrasound images or analysis.

According to an embodiment, storage 260 of system 200 may store one or more algorithms, modules, and/or instructions to carry out one or more functions or steps of the methods described or otherwise envisioned herein. For example, the system may comprise, among other instructions or data, analysis instructions 262 and/or reporting instructions 263.

According to an embodiment, the analysis instructions 262 direct the system to analyze received echo signals. According to an embodiment, the analysis instructions 262 direct the system to analyze each channel individually, find patterns in the echoes that match a model, and select the pattern that best matches the model. According to an embodiment, when analyzing each channel individually, the system can bandpass the signal with 30% bandwidth at a center frequency of the transmit waveform. The system can apply the TGC with the same properties utilized for the main image. The system can detect the waveform using a Hilbert transform or equivalent circuit. The analysis instructions 262 thus direct the system to determine a location of the needle inside the animal or individual relative to the array transducer without requiring steering or focusing of the transmitted beam. The results of the analysis may be utilized immediately, or may be stored in local or remote storage for use in further steps of the method.

According to an embodiment, reporting instructions 263 direct the system to generate and provide to a user via a user interface information comprising the determine location of the needle or other small object. The location information may be provided to a user via any mechanism for display, visualization, or otherwise providing information via a user interface. According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands.

According to an embodiment, within the context of the disclosure herein, aspects of the embodiments may take the form of a computer program product, available from download from a server (e.g. via the internet, or embodied in one or more non-transitory computer readable media having computer readable program code embodied thereon. Thus, according to one embodiment is a non-transitory computer-readable storage medium comprising computer program code instructions which, when executed by a processor, enables the processor to carry out a method including: (i) transmitting, from an element of an array transducer of an ultrasound system, a wide beam; (ii) receiving, at the array transducer, echo signals from the transmitted wide beam, wherein at least some of the echo signals are reflected from a needle located at least partially inside the patient; (iii) analyzing, by a processor of the ultrasound system, the received echo signals; (iv) determining, based on the analysis, a location of the needle inside the patient relative to the array transducer; and (v) reporting, to an ultrasound operator via a user interface, the determined location of the needle inside the patient, comprising overlaying the determined needle location over an ultrasound image of the patient obtained of the determined needle location. The program code may perform entirely on a user's computer, partly on a user's computer, as a stand-alone software package, partly on a user's computer and partly on a remote computer, or entirely on a remote computer or server.

According to an embodiment, the ultrasound system is configured to process many thousands or millions of datapoints to process and analyze received ultrasound echoes and one or more ultrasound images to determine the location of a needle or other small object, as well as to provide a report to the user comprising the location. Indeed, generating this location information is a process that has been relegated to computers because the human mind is incapable or performing the analysis in the timeframe required or with the accuracy required. This requires millions or billions of calculations to generate location information. By providing an improved ultrasound system with improved localization information, the system has an enormous positive effect on patient analysis and care compared to prior art systems.

The present invention may be a system, a method, and/or a computer program product. The computer program product may include a non-transitory computer readable storage medium (or media) having computer readable program instructions thereon for causing a system or processor to carry out aspects of the present invention. The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any combination of the foregoing, among other possibilities. Computer readable program instructions described herein can be downloaded to respective computing/processing devices from a computer readable storage medium or to an external computer or external storage device via a network, for example, the internet, a local area network, and/or a wireless network. Aspects of the present invention are described herein with reference to flowchart illustrations and/or block diagrams of methods, apparatus, systems, and computer program products according to embodiments of the invention. It will be understood that each block of the flowchart illustrations and/or block diagrams, and combinations of blocks in the flowchart illustrations and/or block diagrams, can be implemented by computer readable program instructions.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of ".

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "having," "containing," "involving," "holding," and the like are to be understood to be open-ended, i.e., to mean including but not limited to.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

**Claims**

1. A method (100) for object location using ultrasound, the method comprising:

   transmitting (120), from an element of an array transducer of an ultrasound system (200), a wide beam;
   receiving (130), at the array transducer, echo signals from the transmitted wide beam, wherein at least some of the echo signals are reflected from an object located at least partially inside the patient;
   analyzing (140), by a processor of the ultrasound system, the received echo signals;
   determining (150), based on the analysis, a location of the object inside the patient relative to the array transducer; and
   reporting (160), to an ultrasound operator via a user interface, the determined location of the object inside the patient, comprising overlaying the determined object location over an ultrasound image of the patient obtained of the determined object location.

2. The method of claim 1, wherein overlaying the determined object location over the ultrasound image comprises overlaying an image of the object.

3. The method of claim 1 or 2, further comprising determining (152), based on the determined location of the object inside the patient, that the transducer array is at an improper orientation, and wherein reporting the determined location of the object comprises issuing a warning.

4. The method of any of claims 1 to 3, wherein the location of the object inside the patient is determined in 3D space.

5. The method of any of claims 1 to 4, wherein analyzing the received echo signals comprises a Hough Transform of the received echo signals, and/or comprises a perspective transformation of the received echo signals.

6. The method of any of claims 1 to 5, wherein the object is curved.

7. The method of any of claims 1 to 6, wherein the wide beam is transmitted from a plurality of elements of the array transducer of the ultrasound system.

8. The method of any of claims 1 to 8, wherein the location of the object inside the patient is determined without steering or focusing of a transmitted beam.

9. A computer program product comprising instructions which when executed by an object location system cause the system to carry out the steps of the method of any of claims 1 to 8.

10. A method (100) for guiding an object during an ultrasound of a patient, the method comprising:
    receiving (112), at a user interface of an ultrasound system, a report comprising a determined location of the object inside the patient, wherein the report is generated by:

    transmitting (120), from an element of an array transducer of the ultrasound system (200), a wide beam;
    receiving (130), at the array transducer, echo signals from the transmitted wide beam, wherein at least some of the echo signals are reflected from the object located at least partially inside the patient;
    analyzing (140), by a processor of the ultrasound system, the received echo signals;
    determining (150), based on the analysis, a location of the object inside the patient relative to the array transducer;
    generating (160) a report comprising the determined location of the object inside the patient; and
    adjusting (170) the location of the object in the patient based on the received report.

11. A system (200) for object location during an ultrasound of a patient, the system comprising:

a transducer probe configured to: (i) transmit, from an element of an array transducer, a wide beam; and (ii) receive echo signals from the transmitted wide beam, wherein at least some of the echo signals are reflected from an object located at least partially inside the patient;

a processor (220) configured to: (i) analyze the received echo signals; and (ii) determine, based on the analysis, a location of the object inside the patient relative to the array transducer; and

a user interface (240) configured to report the determined location of the object inside the patient, comprising overlaying the determined object location over an ultrasound image of the patient obtained of the determined object location.

12. The system of claim 11, wherein analyzing the received echo signals comprises a Hough Transform of the received echo signals, and/or comprises a perspective transformation of the received echo signals.

13. The system of claim 11 or 12, wherein the processor is further configured to determine, based on the determined location of the object inside the patient, that the transducer array is at an improper angle, and the user interface is further configured to issue a warning that the transducer array is at an improper angle.

14. The system of any of claims 11 to 13, wherein the wide beam is transmitted from a plurality of elements of the array transducer of the ultrasound system.

15. The system of any of claims 11 to 14, wherein the location of the object inside the patient is determined without steering or focusing of a transmitted beam.

100

Provide an ultrasound system

110

Receive a report of needle localization

112

Transmit a wide beam from a transducer probe of an ultrasound system

120

Receive echo signals

130

Analyze the received echo signals

140

Determine a location of a needle relative to the transducer

150

Determine that array is at an improper orientation

152

Report the determined location of the needle

160

Adjust the location of the needle

170

FIG. 1

FIG. 2

300

330

340

310

330

Linear Array

Wide Transmit Beam

Biopsy Needle

| | | | | | | | | | | | | | | | | 0 | | | | | | | | | | | | | | |

Echo receive beams

350

320

FIG. 3

FIG. 4A

FIG. 4B

## Adjacent element needle depth difference

2deg

20deg

45deg

45deg    20deg    2deg

FIG. 5

Transducer
Array

Array Begin

Array End

610

630

620

Needle

FIG. 6

FIG. 7A

FIG. 7B

FIG. 8

900

FIG. 9

FIG. 10

FIG. 11A

FIG. 11B

FIG. 12A

FIG. 12B

FIG. 12C

FIG. 12D

Europäisches
Patentamt

European
Patent Office

Office européen
des brevets

# EUROPEAN SEARCH REPORT

Application Number

EP 23 16 2534

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | US 2013/274608 A1 (TAKEDA YOSHIHIRO [JP] ET AL) 17 October 2013 (2013-10-17)<br>* abstract *<br>* paragraphs [0007] - [0020], [0066] - [0073], [0076] - [0129], [0152] - [0162], [0171] - [0183]; figures 3-8,13a-b,18a-c,20a-b,21 * | 1-5,7-9, 11-15<br><br>6 | INV.<br>A61B8/08<br>A61B8/00 |
| Y<br><br>A | US 2015/272549 A1 (SAMSET EIGIL [NO] ET AL) 1 October 2015 (2015-10-01)<br>* paragraphs [0034] - [0035], [0039], [0055], [0056]; figures 2,4,6,7,8 * | 6<br><br>5,12 | |
| X | CN 107 080 556 A (SUZHOU QISDA CO LTD; QISDA CORP) 22 August 2017 (2017-08-22)<br>* abstract; figures 1,4 * | 1,11 | |
| A | CN 114 190 983 A (SHENZHEN ADVANCED TECH RESEARCH INSTITUTE OF CHINESE ACADEMY OF SCIENC) 18 March 2022 (2022-03-18)<br>* abstract; figure 1 * | 1-15 | TECHNICAL FIELDS SEARCHED (IPC) |
| A | US 2017/303889 A1 (GRIM KASEY A [US] ET AL) 26 October 2017 (2017-10-26)<br>* paragraphs [0094] - [0095]; figures 16-19 * | 1-15 | A61B<br>G01S |
| A | US 2017/128038 A1 (TSUSHIMA MINEO [JP]) 11 May 2017 (2017-05-11)<br>* paragraphs [0170] - [0200]; figures 18-21 * | 1-15 | |

The present search report has been drawn up for all claims

| Place of search | Date of completion of the search | Examiner |
|---|---|---|
| Munich | 3 September 2023 | Daoukou, Eleni |

EPO FORM 1503 03.82 (P04C01)

## ANNEX TO THE EUROPEAN SEARCH REPORT
## ON EUROPEAN PATENT APPLICATION NO.

EP 23 16 2534

This annex lists the patent family members relating to the patent documents cited in the above-mentioned European search report.
The members are as contained in the European Patent Office EDP file on
The European Patent Office is in no way liable for these particulars which are merely given for the purpose of information.

03-09-2023

| Patent document cited in search report | | Publication date | Patent family member(s) | | Publication date |
|---|---|---|---|---|---|
| US 2013274608 | A1 | 17-10-2013 | JP | 5929368 B2 | 01-06-2016 |
| | | | JP | 2013192627 A | 30-09-2013 |
| | | | US | 2013274608 A1 | 17-10-2013 |
| US 2015272549 | A1 | 01-10-2015 | CN | 106456124 A | 22-02-2017 |
| | | | JP | 6576947 B2 | 18-09-2019 |
| | | | JP | 2017509429 A | 06-04-2017 |
| | | | KR | 20160140858 A | 07-12-2016 |
| | | | US | 2015272549 A1 | 01-10-2015 |
| | | | WO | 2015153189 A1 | 08-10-2015 |
| CN 107080556 | A | 22-08-2017 | NONE | | |
| CN 114190983 | A | 18-03-2022 | NONE | | |
| US 2017303889 | A1 | 26-10-2017 | EP | 3445251 A1 | 27-02-2019 |
| | | | US | 2017303889 A1 | 26-10-2017 |
| | | | US | 2021007708 A1 | 14-01-2021 |
| | | | WO | 2017184805 A1 | 26-10-2017 |
| US 2017128038 | A1 | 11-05-2017 | JP | 6746895 B2 | 26-08-2020 |
| | | | JP | 2017086297 A | 25-05-2017 |
| | | | US | 2017128038 A1 | 11-05-2017 |

EPO FORM P0459

For more details about this annex : see Official Journal of the European Patent Office, No. 12/82